(19) Europäisches Patentamt
European Patent Office
Office européen des brevets

(11) EP 2 381 866 B1

(12) **EUROPEAN PATENT SPECIFICATION**

(45) Date of publication and mention
of the grant of the patent:
**22.03.2017 Bulletin 2017/12**

(21) Application number: **09760319.5**

(22) Date of filing: **10.11.2009**

(51) Int Cl.:
***A61B 34/10*** *(2016.01)*

(86) International application number:
**PCT/IB2009/054993**

(87) International publication number:
**WO 2010/076673 (08.07.2010 Gazette 2010/27)**

(54) **PATH PLANNING FOR REDUCING TISSUE DAMAGE IN MINIMALLY INVASIVE SURGERY**

WEGPLANUNG ZUR REDUZIERUNG DES GEWEBESCHADENS BEI MINIMALINVASIVER CHIRURGIE

PRÉPARATION D'ITINÉRAIRE POUR RÉDUIRE LES DOMMAGES AUX TISSUS EN CHIRURGIE MINIMALEMENT INVASIVE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.12.2008 US 141070 P**

(43) Date of publication of application:
**02.11.2011 Bulletin 2011/44**

(73) Proprietor: **Koninklijke Philips N.V.
5656 AE Eindhoven (NL)**

(72) Inventors:
• **TROVATO, Karen, Irene
Briarcliff Manor, New York 10510-8001 (US)**
• **POPOVIC, Aleksandra
Briarcliff Manor, New York 10510-8001 (US)**

(74) Representative: **van Velzen, Maaike Mathilde
Philips Intellectual Property & Standards
High Tech Campus 5
5656 AE Eindhoven (NL)**

(56) References cited:
**EP-A1- 1 844 725          WO-A1-2008/032230
WO-A2-2007/042986     US-A- 6 112 750
US-A1- 2004 015 070**

**Description**

The present invention relates to a method and a system for computing a path for a minimally invasive device to reach a target while avoiding critical structures and minimizing damage to structures. This path may be used to either control a device (e.g., a bronchoscope or a beveled needle) or may be used to construct a device (e.g., a nested cannula).

Such path planning applications may be performed using the framework taught by Karen I. Trovato, A* Planning in Discrete Configuration Spaces of Autonomous System, University of Amsterdam, 1996.

Specifically, a path planning application must be characterized by a set of key parameters. Each parameter has one or more ranges of valid parameter values that are discretized for easier computer calculation. A combination of all the possible parameter ranges is called the configuration space, and each state of the configuration space provides a unique setting for each of these parameters. Since the configuration space is a discretized space, each state of the configuration space may be considered a 'node' in an N-dimensional graph (frequently N = 2 or 3, but sometimes much higher). 'State' and 'node' are used interchangeably herein.

A neighborhood encapsulates a set of potential transitions based on the core capabilities of the system or device, typically within a certain range. A transition from one state in the configuration space to another 'neighboring' state may be caused by an event or physical motion. The 'neighbors' may also be determined based upon physics or 'rules of the game'. Thus, a neighborhood may include all positions for a knight within one move on an empty chessboard.

Assigned to each transition is a cost imposed for changing between a 'home' state and a neighboring state. Therefore, the combination of the states in configuration space with the neighborhood transitions between them may be imagined as a graph with the states as nodes and transitions as cost-weighted, directed edges.

For many path-planning applications, constraints exist that define illegal states, often because of mechanical limitations, interaction with obstacles, or imposed rules. Thus, there may be identifiable forbidden region(s) of nodes in the configuration space. This can be achieved in many ways. For example, the transitions into a forbidden configuration node may be removed, indicating an illegal move. Alternatively, the nodes may be marked as illegal, or transitions into the node may have infinite (unattainable and high) cost, denoted by $\infty$. Each technique causes the path to avoid obstacles. Illegal states also have a downstream effect. For example, the neighborhood of motions for a car might be an arc of travel forward, in a quarter turn. If a state along the curve is blocked by a corner (an obstacle), then not only is the transition into the corner forbidden, but the transitions beyond the corner are also forbidden.

A 'goal' (or target) position may be mapped to one or more equivalent 'goal' nodes in the discretized configuration space. Multiple 'goal' nodes may exist because the formulation of parameters expressing the system may have more than one solution describing the system 'goal'. For example, both left-handed and right-handed configurations of your arm can reach the same location. The system 'start' is simply transformed to a specific starting node, which is often the current state (i.e. status) of the system or device.

Finding the most desirable series of events leading from a current system node (start) to a desired 'goal' is analogous to finding an optimal path of transitions from the current node to the 'goal' node that incurs a minimum cost while avoiding all illegal nodes. It is also taught that the path may be computed from either the start to the goal, or from the goal to the start. In both cases, the connection of transitions forms a resulting path. Path planning often has a criterion for success sometimes called a space variant metric, a cost metric, or an objective function (e.g., a fastest, shortest, least expensive, etc.). The desirable series of events therefore may be found by planning a path using the configuration space nodes, neighborhood of transitions, costs, forbidden regions, and 'goal', and by defining or setting a 'starting node'. A graph search method such as A* provides an efficient mechanism to determine the optimal path.

WO 2007/042986 A2 discloses a method and system for planning a surgical path whereby free space configuration nodes and forbidden configuration nodes are generated and a path is planned on the basis that these forbidden regions are not entered.

EP 1 844 725 A1 discloses a method for planning the placement of a device in a body, wherein information of at least a part of the internal structure of the body of a patient is analyzed to determine if at least one specific or critical region or structure lies within a trajectory of the device in the body, to a computer program which when running on a computer or loaded onto a computer, causes the computer to perform this method, and to a device for planning the placement of a device in a body comprising an acquisition device for acquiring information of at least a part of the internal structure of the body of a patient and an analyzing device for analyzing the acquired information of the internal structure of the body to determine if at least one specific or critical region or structure lies within a region of interest around the planned trajectory of the device in the body.

As will be further explained herein, the present invention provides a structural damage quantification metric (measure) and a target node geometric expansion that expands the utilization of the subject framework taught by Trovato. For example, a kinematically feasible set of nested cannulas can be computed based on an optimal path for the device that minimizes damage to critical structures during a minimally invasive surgery.

The invention is disclosed in claims 1 and 13. Preferred methods and embodiments are disclosed in the dependent claims. One form of the present invention is a method for planning a path according to a surgical application incorporating a structural damage assessment technique. The method involves a construction of a configuration space structure within a data storage medium, the configuration space structure representing a discretized configuration space of an anatomical region of a body, including free-space configuration nodes and forbidden configuration nodes. The method further involves a generation of a structural damage assessment for each free-space configuration node, the structural damage assessment being indicative of a damage assessment of potential damage to one or more anatomical areas of the anatomical region represented by the forbidden configuration node(s) having an infinite structural assessment cost. The free-space configuration nodes include a safe free-space configuration node having a zero structural damage assessment cost, a risky free-space configuration node having a finite structural damage assessment cost, and a risky free-space configuration node having an infinite structural damage assessment cost. The path planning comprises evaluating a metric representing a cost for transitioning from one configuration node to a neighboring configuration node, wherein the metric depends on the structural damage assessment costs of the free-space configuration nodes.

In a second form of the present invention, the planning method incorporates the geometric expansion technique. Specifically, the method further involves an augmentation of the configuration space node structure as constructed within the data storage medium with parameter values quantifying each node of the configuration space node structure, wherein the augmentation of the configuration space node structure includes a geometric expansion of a target node involving one or more free-space configuration nodes geometrically neighboring the target node serving as surrogate seed node(s). The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims and equivalents thereof.

FIG. 1 illustrates exemplary Brodmann areas of brain as known in the art.

FIG. 2 illustrates an exemplary non-holonomic neighborhood for a nested cannula as known in the art.

FIG. 3 illustrates a block diagram of a critical structure damage assessment technique and a target node geometric expansion technique in accordance with present invention.

FIG. 4 illustrates a flowchart representative of a path planning method in accordance with the present invention.

FIG. 5 illustrates exemplary brain vasculature and ventricles as known in the art.

FIG. 6 illustrates an exemplary detection of critical structures within a brain in accordance with the present invention.

FIG. 7 illustrates a flowchart representative of a weight computation method in accordance with the present invention.

FIG. 8 illustrates an exemplary weighted scale in accordance with the present invention.

FIG. 9 illustrates an exemplary obstacle map in accordance with the present invention.

FIG. 10 illustrates an exemplary dilated obstacle map in accordance with the present invention.

FIG. 11 illustrates an exemplary obstacle distance map in accordance with the present invention.

FIG. 12 illustrates an exemplary empirical weighted map in accordance with the present invention.

FIG. 13 illustrates an exemplary structural damage assessment map in accordance with the present invention.

FIG. 14 illustrates a flowchart representative of a target node geometric expansion method in accordance with the present invention.

FIG. 15 illustrates an exemplary two-dimensional geometric expansion of a target node in accordance with the present invention.

FIG. 16 illustrates a flowchart representative of A* algorithm for determination of an optimal path from a 'seed' node to a 'goal' utilizing state parameters in accordance with the present invention.

FIG. 17 illustrates an exemplary set of "safe" entry points for brain biopsy as known in the art.

FIG. 18 illustrates a block diagram of a system in accordance with the present invention.

The present invention is premised on three (3) primary inventive principles.

First, a discretized configuration space for path planning applications as related to minimally invasive surgeries may be enhanced by creating a 'structural damage assessment' that provides a cost estimate or penalty accrued by traversing specific anatomical areas (e.g., critical anatomical structures within an anatomical region and fragile entry points into the anatomical region). This might be stored in the configuration space itself, or is preferably stored in a separate structure or as a function. The structural damage assessment will facilitate path planning within the anatomical region that minimizes overall damage.

For example, as related to brain surgery, travel through fragile entry points along the skull (e.g., the temple) is undesirable since this path will require subsequent reconstructive surgery. Furthermore, any damage to critical structures of the brain (e.g., blood vessels, ventricles, pituitary glands, pons and optic nerves) may mean loss of life or of key life functions. Even if they are small areas, they could have a very high cost. The utilization of a structural damage assessment aids minimally invasive surgery planning by minimizing, if not preventing, any damage to the critical structures and fragile entry points. As will be further explained, for an A* algorithm, structural damage assessment values may be used in conjunction with the cost metric and heuristic parameter values to search through a discretized configuration space that avoid obstacles, but also maintains a safe spacing from such obstacles.

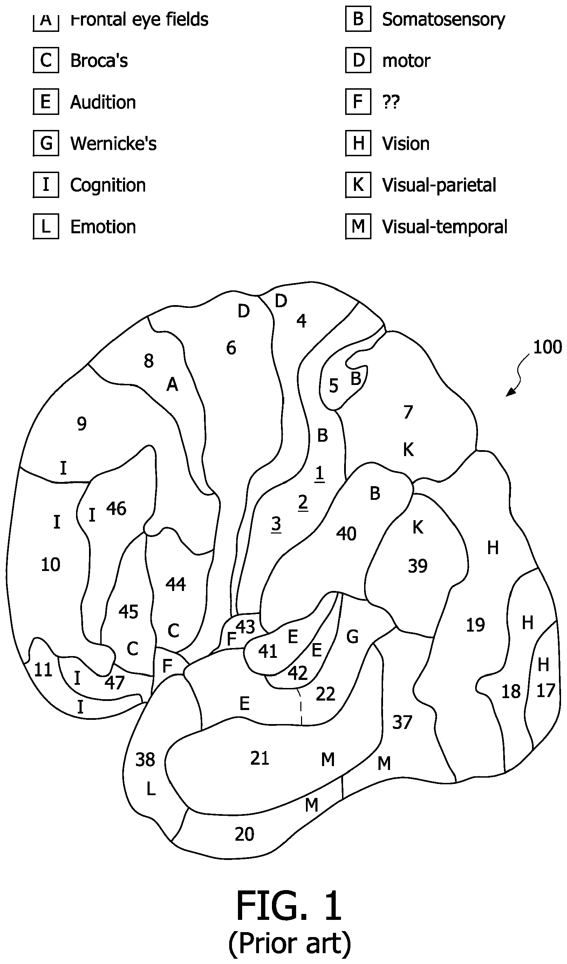

Second, a preferred entry point or area may be highlighted, given the target and structural damage assessment, such as, for example, the Brodmann areas of brain chart 100 shown in FIG. 1. In cases like planning for the brain, where a burr hole may be made in any of numerous locations, it is critical to identify the appropriate starting location based on the target, such that it minimizes overall damage, but is achievable with current tools.

Third, prior path planning applications have been defined for devices including nested cannulas, steerable needles and bronchoscopes. Nested cannulas extend sequentially, from largest to smallest, and are curved in different directions to reach far into the anatomy. The method for configuring these devices is described in International Publication WO 2008/032230 entitled "Active Cannula Configuration for Minimally Invasive Surgery" by Karen I. Trovato. Since nested cannulas do not have motorized joints along their length, nor 'marionette wires' for control, they may be made very small, which is useful for brain applications as well as many other minimally invasive procedures. The steering of other devices such as a bronchoscope and beveled needle is described in International Publication WO 2007/042986 entitled "3D Tool Path Planning, Simulation and Control System" of Karen I. Trovato et al. For some devices and applications, the approach orientation to a target may not be easily identifiable or selectable.

A common problem is how to create multiple approach orientations to a target, for example, the center of a tumor, without tedious manual entry, while maintaining a 3D configuration space for 6D planning. In a simple example, the approach orientation may be formed by the direction from adjacent neighbors to a seed node. The search direction is therefore set to the opposite direction (from seed to adjacent neighbors). This "geometric expansion" provides a simple, achievable variety of orientations while covering all surrounding directions. If these neighbors are within acceptable accuracy to represent the target, they may be used as surrogate 'seeds', having zero cost and the defined orientation for initiating the A* search, as long as they are not in a forbidden or infinite cost location. Clearly, geometric neighborhoods of many shapes and sizes may be used, not just those that are immediately adjacent, however they must define viable final motions or actions. Finally, the geometric neighborhood used to set the orientation of surrogate seeds does not necessarily have to match the neighborhood used during the A* search.

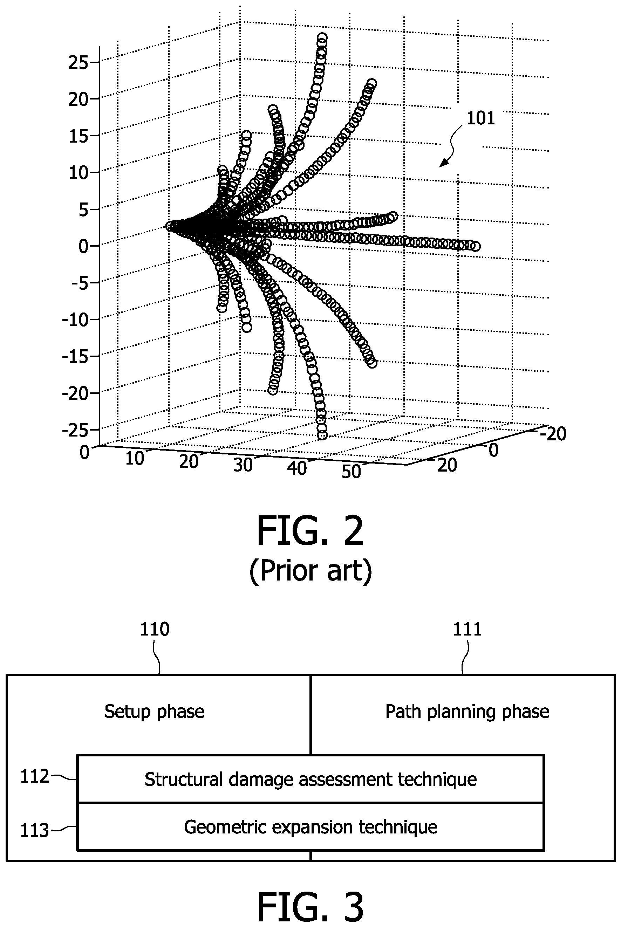

For example, FIG. 2 illustrates a non-holomonic neighborhood 101 of arcs that may be utilized to compute a series of nested cannulas to reach a target location within an anatomical region of a body as known in the art. A search of neighborhood 101 is typically expanded in an A* algorithm based on a single orientation leading into a single target node. However, a geometric expansion of the present invention would facilitate a search of neighborhood in an A* algorithm based on multiple orientations surrounding a single target node. The result may be a curved route through the anatomical region leading between the target node and the optimal, permitted entry point. Those having ordinary skill in the art will appreciate that this geometric expansion in conjunction with the aforementioned structural damage assessment provides for the generation of a kinematically feasible path for devices (e.g., a bronchoscope or a beveled needle), and for the construction of a kinematically feasible nested cannula that minimizes damage to sensitive structures or fragile entry points during a minimally invasive surgery.

It is to be understood by persons of ordinary skill in the art that the following description of FIGS. 3-18 are provided for purposes of illustration of the aforementioned inventive principles of the present invention in general terms with specific yet straightforward examples and not for limiting the practice of such inventive principles. In particular, unnecessary detail of known functions and operations may be omitted from the description of the inventive principles herein so as not to obscure the present invention. Nonetheless, an artisan will understand how to practice the inventive principles of the present invention to any type of path planning application (i.e., surgical tool path planning, vehicle path planning, economic system path planning, etc.) and will further understand that there are many variations that lie within the scope of the appended claims.

FIG. 3 illustrates a setup phase 110 and a path-planning phase 111 for any type of path planning application, in particular for planning of a surgical path for an instrument within a patient during a minimally invasive surgery or for planning a construction of a nested cannula. In general terms, setup phase 110 may minimally involve (1) a construction of a configuration space node structure representing a discretized configuration space including a plurality of states (nodes) characterized by one or more parameters, (2) an identification of a neighborhood encapsulating all of the allowed actions that cause changes or transitions between states (nodes) in the discretized configuration space, and (3) a formulation of a metric representing the cost for transitioning from one state to a neighboring state as defined by the 'neighborhood'. Furthermore, in general terms, path planning phase 111 may minimally involve (1) an identification or definition of a seed node within the discretized configuration space, and (2) a utilization of the seed node to initiate a propagation of cost waves through the configuration space node structure based on the metric to find the most desirable series of events between a start node and a goal node.

The present invention introduces a structural damage assessment technique 112 and a geometric expansion technique 113 that may individually or collectively be incorporated in setup phase 110 and path planning phase 111 of the path planning application. In general terms, a configuration space node structure includes a plurality of nodes with each node being at a different discrete location in the discretized configuration space as characterized by the parameter(s), and technique 112 provides for the use of structural damage assessment values explicitly quantifying a damage assessment of potential damage to anatomical structures of the anatomical region of the body while technique 113 provides for the use of one or more geometrically neighboring free-space neighbors of a target node as surrogate seed nodes during an execution of a search through the free-space of the discretized configuration space.

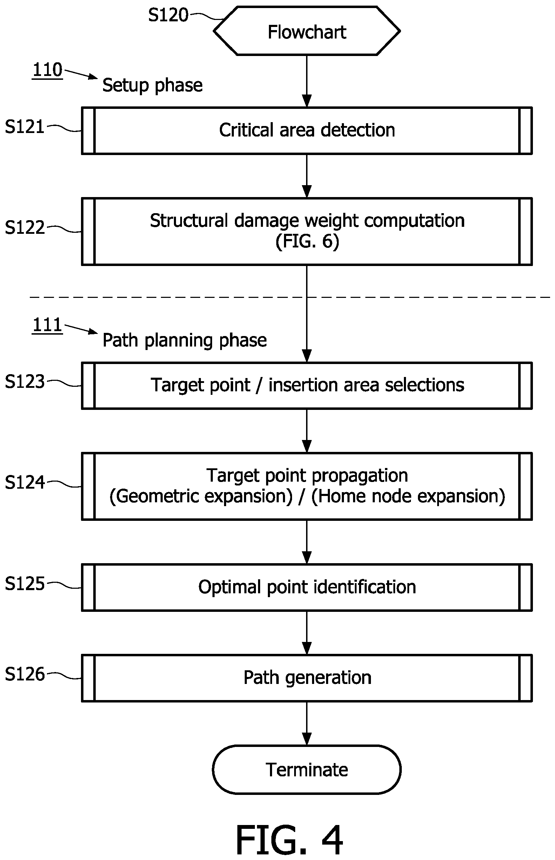

Exemplary embodiment of techniques 112 and 113 as shown in FIGS. 4-18 will now be described for the purpose of facilitating a further understanding of the inventive principles of the present invention whereby those having ordinary skill in the art will appreciate the various benefits of the present invention.

A. PATH PLANNING METHOD INCORPORATING A

STRUCTURAL RISK ASSESSMENT AND GEOMETRIC EXPANSION

FIG. 4 illustrates a flowchart 120 representative of a path planning method of the present invention. The objective of this method is to acquire a practical set of cost values, of the states of a discretized configuration space during setup phase 110 (FIG. 3) of a path planning application to facilitate an optimal search during path planning phase 111 (FIG. 3) of the path planning application. This objective is subsequently described herein in the context of an incorporation of the structural damage assessment technique 112 and the geometric expansion technique 113 of the present invention. Thus, a description of well-known path planning processes executed during phases 110 and 111 is provided only as needed to facilitate an understanding of the techniques 112 and 113 of the present invention.

Referring to FIG. 4, setup phase 110 includes a stage S121 and a stage S122 of flowchart 120.

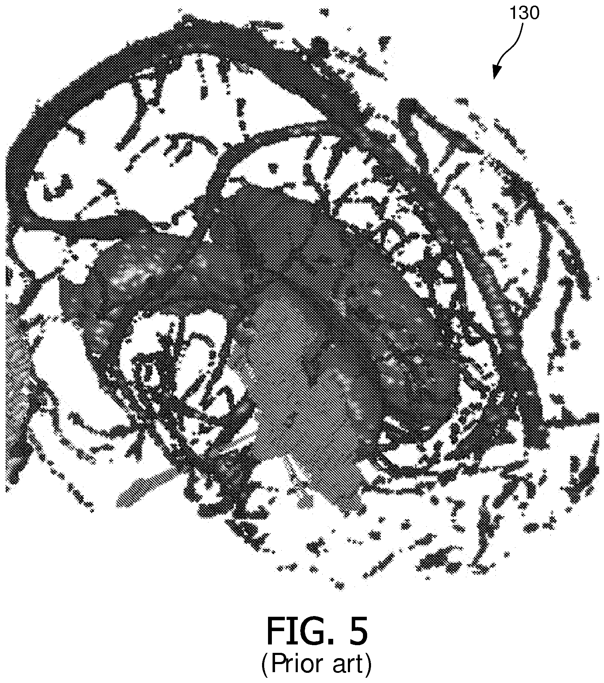

Stage 121 encompasses a detection of anatomical areas within an anatomical region of a body for which it is critical to minimize or prevent any damage to the structures. These critical areas include, but are not limited to, fragile entry points into the anatomical region and fragile structures within the anatomical region susceptible to damage from an instrument used during a minimally invasive surgery in the region, such as, for example, the brain vasculature/brain ventricles 130 shown in FIG. 5. In one embodiment of stage S121, a detection of critical areas may be achieved from images of the anatomical region on a manual basis or an atlas based basis. For example, a manual based detection may involve a computed tomography, a magnetic resonance or the like image that is manually segmented or with known semi-automatic algorithms in view of a moderate number of critical structures that are simple to outline.

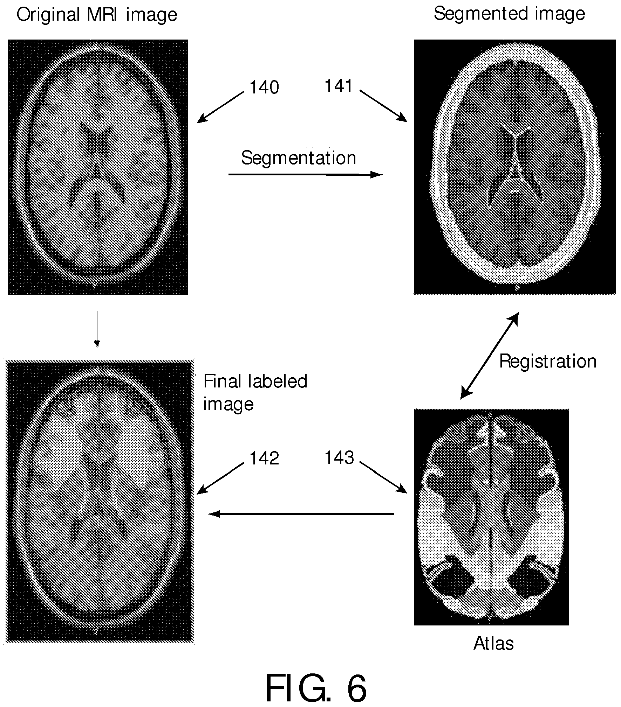

Conversely, for critical areas not detectable from imaging, a registration between segmented patient specific data and an anatomical/functional atlas may be used. FIG. 6 illustrates an exemplary segmentation and registration of a slice of an image of a brain. Specifically, in brain surgery, an MRI is usually used for both diagnostics and planning. An automatic algorithm or manual segmentation can detect major tissue types, such as, for example, white mater, grey mater, dura mater, blood vessels, cerebrospinal fluid, skull, and skin. However, for a minimally invasive path, other structures, not visible in MRI, such as Brodmann areas 100 as shown in FIG. 1, may be used to define structural damage causing functional impairment. To this end, a manual or (semi)-automatic segmentation of visible areas within a cross-sectional MRI image 140 of a human brain results in a segmented image 141 that is registered in a deformable manner to a known detailed cross-sectional atlas 143 of the human brain to yield a labeled image 142. One such known atlas is Talairach atlas (ref: Lancaster JL, Woldorff MG, Parsons LM, Liotti M, Freitas CS, Rainey L, Kochunov PV, Nickerson D, Mikiten SA, Fox PT, "Automated Talairach Atlas labels for functional brain mapping". Human Brain Mapping 10:120-131, 2000), a set of 1004 different neurological labels, labeling every pixel of the brain atlas. If a registration between the atlas 143 and segmented image 141 is performed, a transformation between each voxel in the patient's image 141 and each voxel of the atlas model 143 is established. Therefore, each element of patient's image 141 is labeled based on its neurological function. Non-neurological structures (e.g., CSF or blood vessels) are labeled in the segmentation process.

Stage S122 encompasses a structural damage assessment for each free-space configuration node representing non-critical areas of the anatomical region as being safe or risky. In general terms, the cost of each forbidden configuration node representing the detected critical areas (obstacle and fragile entry points) of the anatomical region corresponds to a critical anatomical area having an infinite risk cost. Conversely, the risk cost of each safe free-space configuration node has a zero risk of structural damage while each risky free-space configuration node has an estimated risk of structural damage ranging from a non-zero, finite cost to an infinite cost. In summary, a forbidden configuration node cost is $C = \infty$, a safe free-space configuration node cost is $C = 0$, and a risky free-space configuration node cost is $C \leq \infty$.

In one embodiment of stage S122, the costs associated with each free-space configuration node, may be set by a user, derived from forbidden configuration nodes or a combination of individual set costs and the forbidden configuration nodes, or may be set by a computer program, for example from an automatic segmentation process. For example, for a neurosurgical application, joint costs might be formed by combining (e.g. adding or averaging): a) a cost for nearby critical points or areas (e.g. distance from critical anatomical areas) and b) a non-forbidden, risky cost.

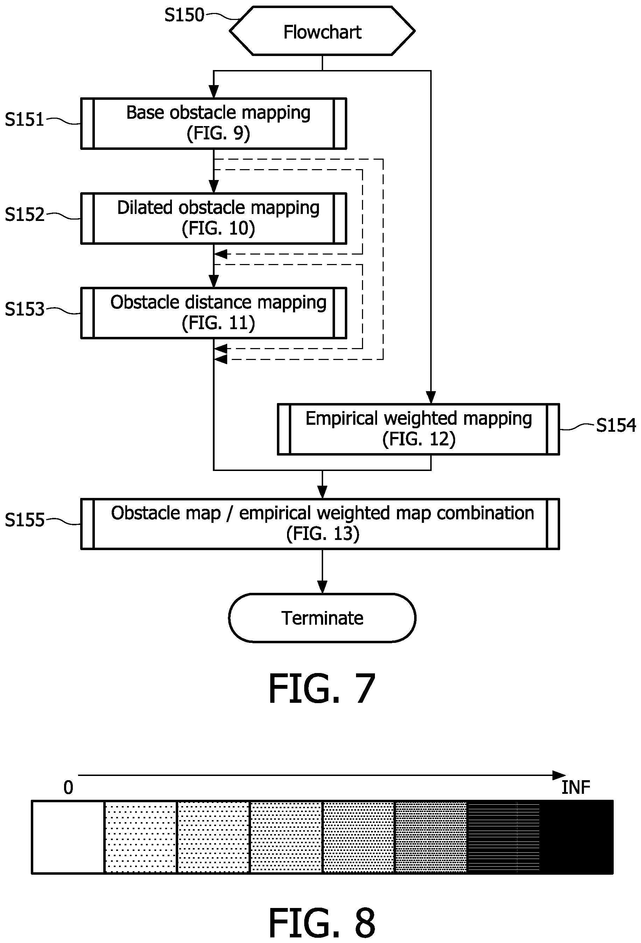

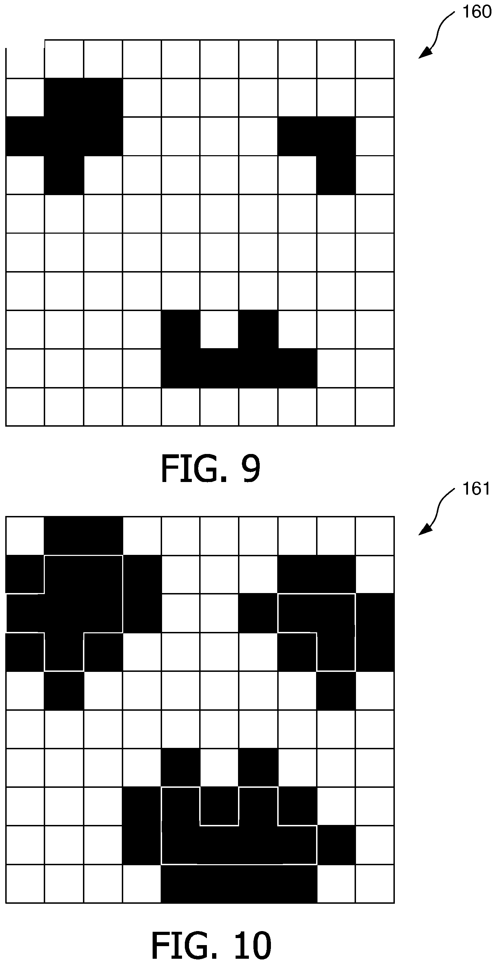

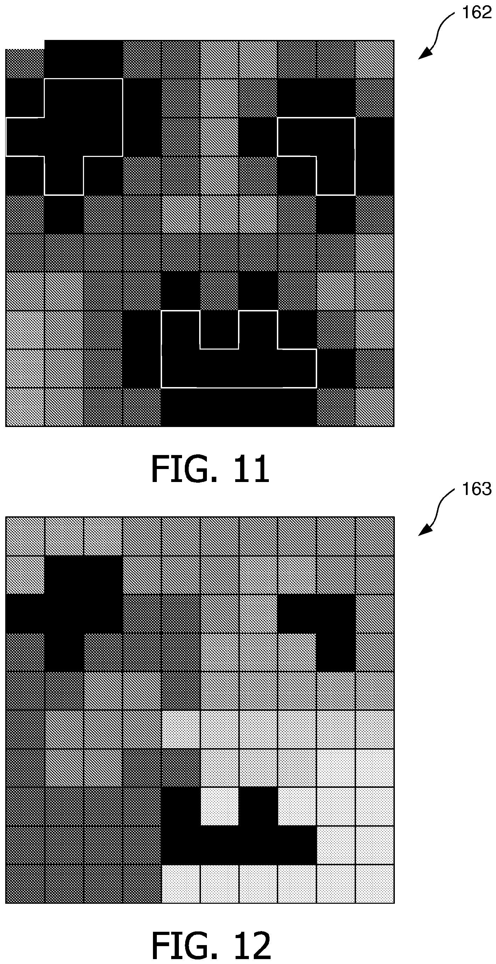

FIG. 7 illustrates a flowchart 150 representative of a cost computation method of the present invention that will be described as a simplified 2D example of labeled image (e.g., labeled image 142 shown in FIG. 6) and a colored coded cost scale shown in FIG. 8 ranging from a zero white coded cost to an infinity black coded cost. A stage S151 of flowchart 150 encompasses a generation of a base obstacle map derived from forbidden configuration nodes of a labeled image, such as, for example, an obstacle map 160 shown in FIG. 9 having infinity black colored nodes associated with forbidden areas. A stage S152 of flowchart 150 encompasses a generation of a dilated obstacle map derived from safety zones established around the forbidden areas. For example, a dilated obstacle map 161 shown in FIG. 10 having infinity black coded safety nodes associated with the forbidden areas has an additional buffer of infinite cost free-space configuration nodes surrounding the original forbidden configuration nodes. The white edges around the original forbidden configuration nodes are only to help visually separate the newly added infinite cost free-space configuration nodes from the original forbidden configuration nodes. A stage S153 of flowchart 150 encompasses a generation of an obstacle distance map derived from the distance to the nearest infinite cost areas. For example, an obstacle distance map 162 shown in FIG. 11 has finite grey colored nodes decreasing in value as free-space configuration nodes are spaced farther from any buffered critical zone.

A stage S 154 of flowchart 150 encompasses a generation of an empirical data map from the labeled image with the empirical data being indicative of the physical sensitivity of each critical anatomical area (obstacles and fragile entry points) to an external stimuli (surgical instruments/tools). For example, an empirical weight map 163 shown in FIG. 12 having zero or finite grey colored nodes corresponding to the physical sensitivity at each of the free-space configuration nodes as empirically ascertained by a user or an automatic segmentation process.

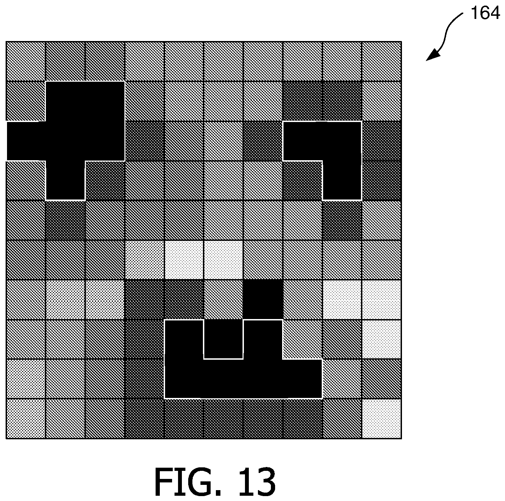

A stage S155 of flowchart 150 encompasses a combination of the obstacle distance map and the empirical data map based on a combination (e.g., a summation and/or an averaging) of matching nodes. For example, in FIG. 13, a cost map 164 has color coded areas with values collectively derived from the forbidden areas and empirical data in a manner to avoid the forbidden areas to the greatest extent possible to thereby minimize, if not prevent, any structural damage to anatomical structures associated with the forbidden areas.

While those having ordinary skill in the art will appreciate flowchart 150 as shown will facilitate a very high safety to forbidden points and areas, the following is a description of alternative embodiments of flowchart 150 that may be implemented in practice.

In a first alternative embodiment of flowchart 150, dilated obstacle mapping stage S 152 may be omitted as indicated by the dashed arrow leading from base obstacle mapping stage S 151 to obstacle distance mapping stage S 153.

In a second alternative embodiment of flowchart 150, obstacle distance mapping stage S153 may be omitted as indicated by the dashed arrow leading from dilated obstacle mapping stage S 152 to mapping combination stage S155.

In a third alternative embodiment of flowchart 150, both dilated obstacle mapping stage S152 and obstacle distance mapping stage S153 may be omitted as indicated by the dashed arrow leading from base obstacle mapping stage S 151 to mapping combination stage S 155.

In a fourth alternative embodiment of flowchart 150, obstacle mapping stages S151-S153 may be omitted whereby empirical weighted mapping stage S 154 is exclusively utilized for structural damage assessment cost computation.

In a fifth alternative embodiment of flowchart 150, empirical weighted mapping stage S 154 may be omitted individually or with one or more of the obstacle mapping stages S151-S153 whereby the remaining obstacle mapping stage(s) is(are) utilized for structural damage assessment cost computation.

Referring again to FIG. 4, path planning phase 111 includes stages S123-S126 of flowchart 120.

Stage S123 encompasses a user or computer identified target point, such as a tumor centroid, and a user or computer-identified set of one or more acceptable tool-insertion points/areas for the applicable surgical procedure. For example, a single insertion area for a surgical instrument (e.g., a nested cannula) in the lung might be the cross-section of the trachea at a specific CT slice. Alternatively, one or more insertion areas may be selected for entrance of the surgical instrument from any non-fragile area of the skull. A viable path only exists if it is possible to reach from the entrance to the target, with acceptable overall cost. A physician, who weighs the risk and the benefit, must determine the limits of 'acceptability'.

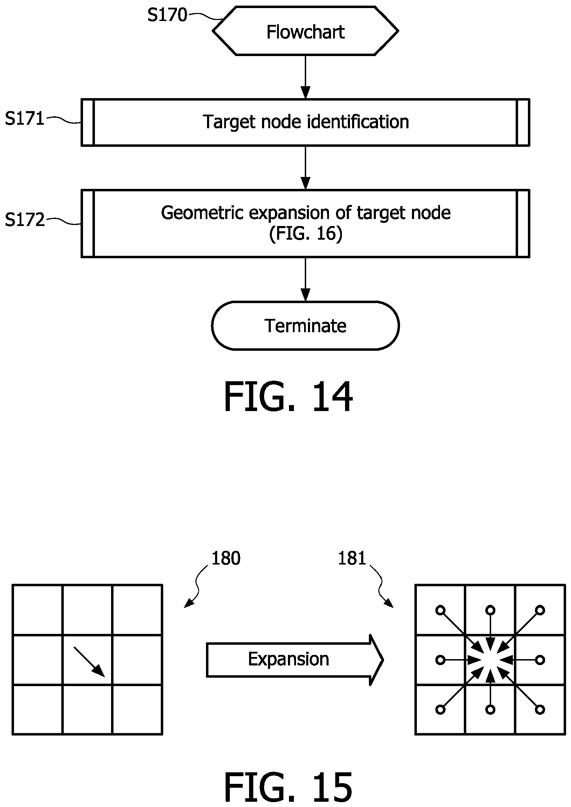

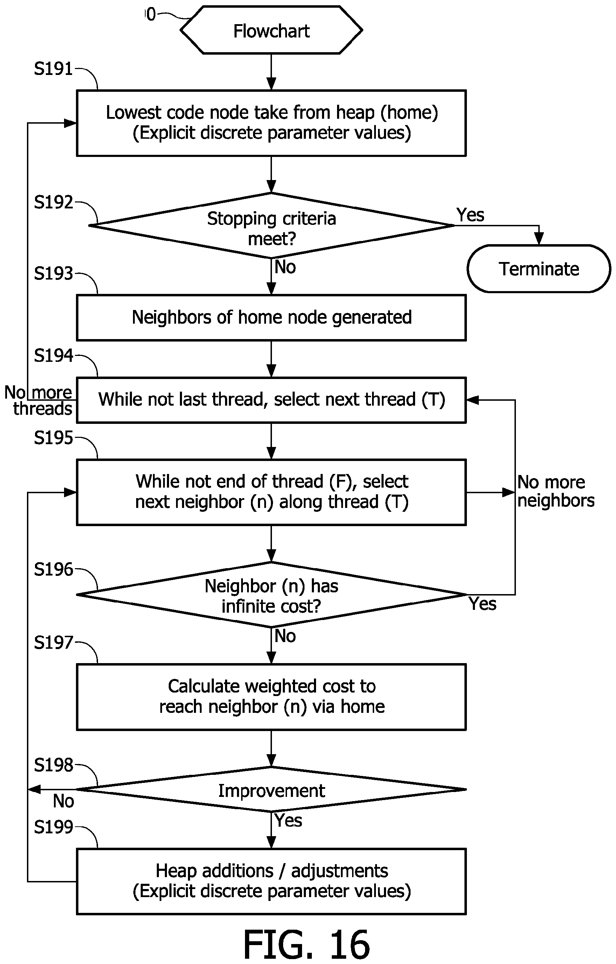

Stage S124 encompasses a propagation from the target point to the insertion point(s) and/or insertion area(s) via an A* algorithm. In one embodiment of stage S124, a flowchart 170 as shown in FIG. 14 and a flowchart 190 as shown in FIG. 16 are executed.

Referring to FIG. 14, flowchart 170 is representative of a geometric expansion method of the present invention. A stage S 171 of flowchart 170 encompasses an identification of a target node corresponding to the selected target point within the anatomical region, and a stage S172 of flowchart 170 encompasses a geometric expansion of the target node to identify geometrically neighboring free-space configuration nodes. One or more of these geometrically neighboring free-space configuration nodes may serve as surrogate seed nodes, with orientation set by the angle formed between the target node and geometrically neighboring free-space configuration node in an execution of flowchart 190 as will be subsequently explained herein.

FIG. 15 illustrates a standard initial condition 180 of the centered target node in a simplified two-dimensional ("2D") space that is defined by a point and an orientation (e.g., -50°, 0°, 0°). Alternatively, the target node may be replaced by the set of geometrically neighboring free-space configuration nodes, where each node points outward to set the direction of the search that is transformed to an expanded initial condition 181 of the centered target node having each geometrically neighboring free-space neighbor defined by a point, an orientation, a thread and a cost.

Referring to FIG. 16, flowchart 190 is representative of A* algorithm for determination of an optimal path from each expanded 'seed' node to a insertion point/area 'goal' utilizing implicit or preferably explicit discrete parameter values based on a neighborhood 101 as shown in FIG. 2. For the geometric expansion technique, each 'seed' node is a free-space surrogate node geometrically neighboring the target node having a structural damage assessment cost below infinity or a critical damage threshold less than infinity.

In execution, a first expanded 'seed' node is placed into the heap in order to begin cost wave propagation, or A*. The heap is a balanced binary tree that maintains the lowest cost value at the root. The lowest cost node taken from the heap is called 'home', in step S 191 of flowchart 190. In earlier robotics and path planning applications, the path was often generated using goals as the seed nodes. In those applications, the cost of a node (g(n), described later) was called 'cost_to_goal'. In this application, we revise the terminology to the more general 'cost_to_seed', but they should be considered equivalently. There are well- known algorithms for managing sort structures, including heaps.

Step S 191 further encompasses the acquisition of the detailed configuration space information of the 'home' node, preferably the explicit discrete parameter values of the home node. This will provide the precession desired for the cost wave propagation without any negative impact on the speed and memory capacities of a system executing flowchart 190.

A step S 192 of flowchart 190 encompasses a testing of a "stopping criterion". There are many tests that may be performed to determine if the process may stop. The "stopping criterion" may include, but not limited to, (1) a test of whether the heap is empty and (2) a test of whether the current ('home') node's is one of identified insertion points or belongs to one of insertion areas. This stopping criterion assures that there a viable connection between the insertion and target, and that value is a minimum for that location. This enables the search to terminate before the entire space is filled, yet nonetheless it does give the optimal path between the 'start' and 'goal'. A third stopping criterion customized for the present invention involves a presentation of a colored coded surface of a reached insertion point or insertion area previously selected by a user based on the cost to reach the insertion point or insertion area whereby a selection of an entrance point by a user is considered a selection of an optimal path between the 'start' and 'goal'.

If the 'stopping criterion' is met, then flowchart 190 is terminated. Otherwise, if the "stopping criterion' is not met, then a step S 193 of flowchart 190 encompasses a generation of the neighborhood of permissible transitions. The neighbors of the home' node are calculated based on the 'home' node's orientations given by its alpha, theta and phi as well as its 'home' x, y, z location. The neighborhood results from rotating the nominal neighborhood by alpha, theta and phi, and then translating the already rotated neighborhood relative to the 'home' node's x, y, z location. Methods for rotation and translation of points are well known to those skilled in the art.

The resulting neighborhood is then translated and rotated to the location of the current expanding node.

Once the neighbors for the current'home' node are computed, flowchart 190 proceeds to a step S 194 where the next thread (T) of the neighborhood is chosen, if any. If there are no more threads, then flowchart 190 returns to step S 191. Otherwise, if there is a thread (T), then flowchart 190 proceeds to a step S 195 to chose next neighbor (n') along the thread (T) if any. The position and orientation of neighbor n' is computed relative to the 'home' node's position and orientation for the current given thread.

If there are no more neighbors along this thread (T), then flowchart 190 returns to step S 194. If there is another neighbor (n), then flowchart 190 proceeds to a step 196 to test the cost value of the neighbor. If the cost is infinite, or there is another indication that the neighbor is not passable, then flowchart 190 returns to step S 194. Another indication might be that the neighbor has a cost value higher than some pre-determined threshold, which is less than infinity, but too high to pass. This threshold may be a function of the current distance traveled (at the 'home' node), for example.

If the neighbor does not have infinite cost and is passable, the flowchart 190 proceeds to step S 197 to calculate the proposed new cost g(n') for the new neighbor, n'. In the A* algorithm, two costs are computed. The first is called 'g(n')'. This is the cost of the best path (so far) to node n', that arrives from a 'home node' or 'parent node', often denoted as n, without a prime ('). The function g(n') includes the cost to reach the home node, plus the transition cost from the home node to n', plus any structural damage that might arise from transitioning from home to n'. When planning a path for the nested cannula, the length of the path is often the transition cost. The structural damage incurred from travelling from home to n' may be calculated as the sum of each interim state's structural damage cost, counting states beyond n (since n has already been counted), through and including n'. This can also be computed in an alternative way, where the overall structural damage considers the size of the tube as it traverses each voxel (volume element), such that the integral volume swept by the tube is weighted by the various structural damage regions, for example. Therefore, a 3mm diameter tube may be expected to generate twice the damage of a 1.5mm diameter tube. Another alternative may occur when the over-riding cost is the total structural damage regardless of distance. In this case, the transition cost may be only an estimate of structural damage with no cost incurred for distance.

The second value computed in A* is f(n'), which in simplest terms is the 'best case scenario' for a path that first travels through 'home' (by whichever path it arrives from the seed), then travels through n' (including penalties), and finally proceeds optimistically (directly) to the entry point using a heuristic function, h(n'). As is known to those skilled in the art, there are many possible heuristics. An optimistic cost for the heuristic might be zero, for example, however this uninformed heuristic does not provide guidance for the search. Another commonly used heuristic is the straight-line, or Euclidean distance 'as the crow flies', or in other words, the 'distance to go'. Yet another estimate might be the total structural damage cost over the remaining distance with the current sized tube. By choosing a heuristic that is optimistic, the overall cost of f(n') then represents the net desire-ability of a path that goes through n'. In summary,

$$g(n') = g(\text{'home'}) + \text{transition}(\text{'home'}, n') + C(\text{'home'}, n')$$

$$f(n') = g(n') + h(n')$$

The costs for g(n') and f(n') are often stored with other data for node n'.

Flowchart 190 thereafter proceeds to a step S 198 to compare the newly calculated cost F(n') with the pre-existing cost at n', F(n'). If the newly calculated F(n') is greater than pre-existing cost F(n'), then it is more costly to reach n via the 'home' node than whatever was determined previously (i.e., there is no improvement), and flowchart 190 returns to step S195. If the calculated cost, F(n') is less than pre-existing cost F(n'), then this value is an improvement over the prior directions whereby flowchart 190 proceeds from step S 198 to step S 199. Step S 199 adds the neighbor node to the set of possible nodes for opening (expanding) next. This set may be stored in a heap, for example, and the set sorted via a heap sort. If the neighbor node is already on the heap, then the values of the node are updated and the heap is re-sorted. The node x, which has the lowest cost f(x) therefore identifies the most desirable node to explore (expand) next.

Step S 199 further encompasses assigning the new cost_to_seed to n, as well as the specific position (e.g. x, y, z) and orientation (e.g. alpha, theta and phi). In a 6D space, position and orientation are represented with a 3D position (x,y,z) and 3 angles (alpha, theta, phi). If a set of permissible motions is captured in a neighborhood structure with discrete angular displacement (for example FIG. 2) the problem is reduced to 5D, since the $6^{th}$ dimension is captured by the neighborhood. Therefore, a pose is defined with 3D position (x,y,z) and 2 angles (theta, phi). The revised vector is assigned a pointer to 'home', because it leads the best way to the 'seed' node. Optionally, but preferably, the number and type of the thread is also stored. This is used to determine tube dimensions for avoiding obstacles, as well as curvature options. Further, the thread number maps directly to the control parameters used for controlling devices such as a bronchoscope, or maps directly to the selection of tube and its relative orientation to adjacent nested tubes.

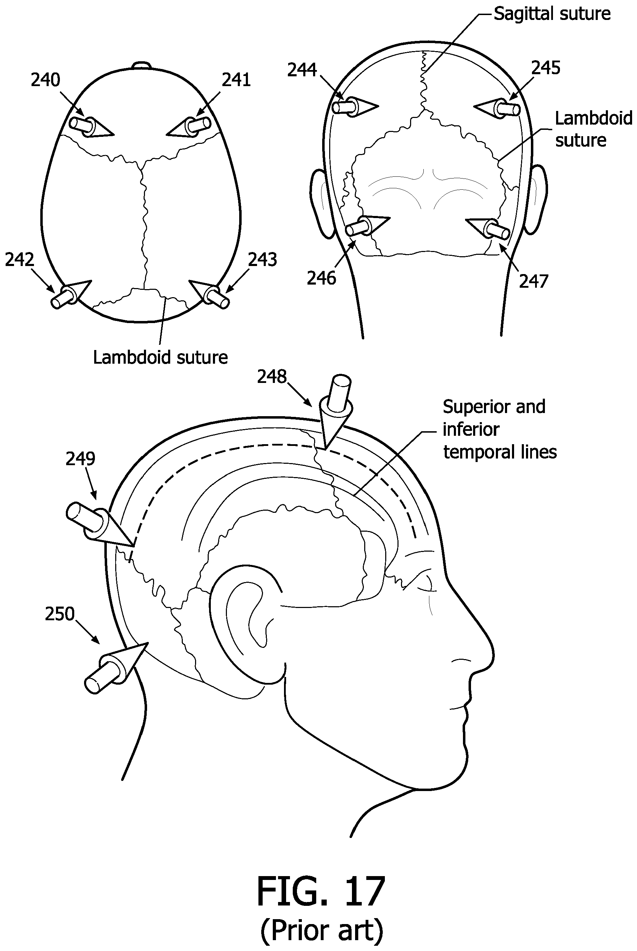

Referring back to FIG. 4, stage S125 encompasses providing a set of entry locations, including special identification of an optimal location so that a doctor can select a preferred entry point such as, for example, the entry points 240-250 as shown in FIG. 17 and taught by Set of typical "safe" entry points for brain biopsy. Source: Sekhar, Fessler. Atlas of Neurosurgical Techniques. Chapter 33: Stereotactic Biopsy (Schwartz and Sisti), pp. 422-429. For example, the planned path may provide between a target and 4 equivalent minimum-cost entry points on the skull. There also may be 10 other locations highlighted, along with their respective higher costs. The doctor may choose one of these points, which must be on a suitable stopping location or subject to a stopping criterion.

Based on the selected point, the path will be extracted that determines the set of control parameters, or the configuration of the device, or the intended path from the selected point to the target. Optionally, this path may be presented within a 3D image of the anatomical region during stage S126.

B. PATH PLANNING SYSTEM INCORPORATING A STRUCTURAL RISK ASSESSMENT AND/OR GEOMETRIC EXPANSION

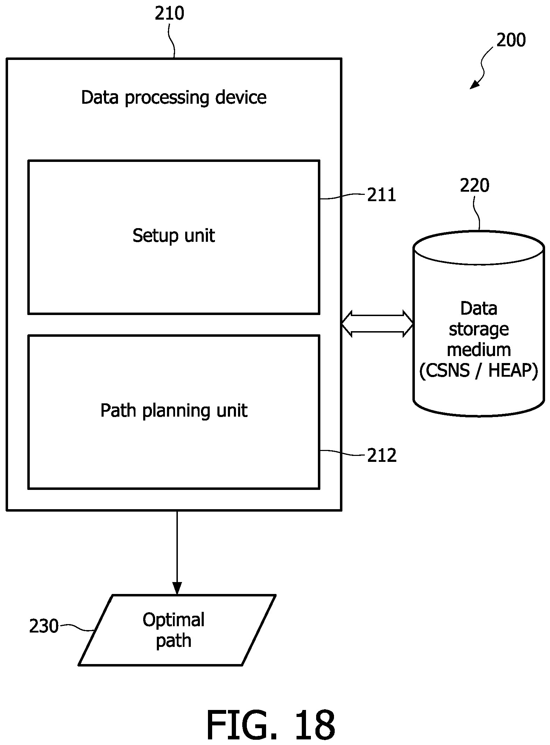

Referring now to FIG. 18, a system 200 is illustrated for a path planning application in accordance with the present invention. The system 200 includes a data processing device 210 and a data storage medium 220. Data processing device 210 employs a setup unit 211 and a planning unit 212 as physically separate or integrated units for implementing the structural damage assessment technique and/or geometric expansion technique of the present invention as previously explained herein in connection with FIGS. 3-17. In general terms, setup unit 211 performs all tasks necessary to construct a configuration space data structure ("CSDS") and other structures/functions appropriate for the particular path planning application within data storage medium 220 of any type (e.g., RAM), and planning unit 212 propagates cost waves as needed to fill the configuration space node structure with costs values as a function of the parameter values in accordance with the structural damage assessment and/or geometric expansion features of the present invention as desired for the particular path planning application. The result is an optimal path 230 in a format suitable for the particular path planning application.

The method or system are used to create a path. This path may be used in several ways. The path can either control a device, such as a bronchoscope or beveled needle. Alternatively, it may be used to construct a device such as a nested cannula. Finally, it may be displayed on a screen, or superimposed in a 3D image and displayed within 3D goggles.

While various embodiments of the present invention have been illustrated and described, it will be understood by those skilled in the art that the methods and the system as described herein are illustrative, and various changes and modifications may be made and equivalents may be substituted for elements thereof without departing from the true scope of the present invention. In addition, many modifications may be made to adapt the teachings of the present invention to entity path planning without departing from its central scope. Therefore, it is intended that the present invention not be limited to the particular embodiments disclosed as the best mode contemplated for carrying out the present invention, but that the present invention include all embodiments falling within the scope of the appended claims.

**Claims**

1. A method (120) for planning a path according to a surgical application, the method comprising:

> (110) constructing a configuration space node structure within a data storage medium (220), the configuration space node structure including free-space configuration nodes and forbidden configuration nodes representing a discretized configuration space of an anatomical region (100) of a body, and
> (S122) generating a structural damage assessment for each free-space configuration node, the structural damage assessment being indicative of an assessment of potential damage to at least one critical anatomical area of the anatomical region (100) represented by the forbidden configuration nodes, each forbidden configuration node having an infinite structure damage assessment cost,
> wherein the free-space configuration nodes include: a safe free-space configuration node having a zero structural damage assessment cost; a risky free-space configuration node having a finite structural damage assessment cost; and a risky free-space configuration node having an infinite structural damage assessment cost,
> wherein the path planning comprises evaluating a metric representing a cost for transitioning from one configuration node to a neighboring configuration node, wherein the metric depends on the structural damage assessment costs of the free-space configuration nodes.

2. The method of claim 1, wherein the least one critical anatomical area includes at least one of:

> a critical anatomical structure within the anatomical region (100); and
> a fragile entry point into the anatomical region (100).

3. The method of claim 1, wherein the generation of a structural damage assessment for each free-space configuration node includes:

> generating a labeled image (142) of the anatomical region (100) including at least one forbidden area of the anatomical region (100); and
> computing structural damage assessment costs for each free-space configuration node associated with the labeled image (142).

4. The method of claim 3, wherein the generation of the labeled image (142) of the anatomical region (100) includes:

> registering at least one patient image of the anatomical region (100) with an atlas (143) of the anatomical region (100).

5. The method of claim 3, wherein the computation of structural damage assessment costs for each free-space configuration node associated with the labeled image (142) includes:

> generating a base obstacle map (160) derived from each forbidden area of the anatomical region (100) associated with the labeled image (142), each free-space configuration node of the base obstacle map (160) having a structure damage assessment cost less than infinity.

6. The method of claim 5, wherein the computation of structural damage assessment costs for each free-space configuration node associated with the labeled image (142) further includes:

generating a dilated base obstacle map (161) derived from at least one safety zone established around each forbidden area of the anatomical region (100), each free-space configuration node of the dilated base obstacle map (161)within one of the at least one safety zone having an infinite structure damage assessment cost.

7. The method of claim 3, wherein the computation of structural damage assessment costs for each free-space configuration node associated with the labeled image (142) further includes:

generating an obstacle distance map (162)derived from a distance to a nearest forbidden area of the anatomical region (100).

8. The method of claim 3, wherein the computation of structural damage assessment costs for each free-space configuration node associated with the labeled image (142) further includes:

generating an empirical weighted map (163) derived from an empirical physical sensitivity of each forbidden area of the anatomical region (100) associated with the labeled image (142).

9. The method of claim 1, further comprising:

augmenting the configuration space node structure as constructed within the data storage medium (220) with structural damage assessment values quantifying each node of the configuration space node structure.

10. The method of claim 9, wherein the augmentation of the configuration space node structure includes a cost function of a total distance of cumulative transitions between nodes along the path.

11. The method of claim 9, wherein the augmentation of the configuration space node structure includes a cost function of an instrument associated with the surgical application involving of at least one dimension of the instrument.

12. The method of claim 9, wherein the augmentation of the configuration space node structure includes a geometric expansion of a target node involving at least one free-space configuration node geometrically neighboring the target node serving as a surrogate seed node.

13. A system for planning a path according to a surgical application, the system comprising:

a data storage medium (220); and
a data processing device (210) in electrical communication with the data storage medium (220) to construct a configuration space node structure within the data storage medium (220), the configuration space node structure including free-space configuration nodes and forbidden configuration nodes representing a discretized configuration space of an anatomical region (100) of a body,
wherein the data processing device (210) is operable to generate a structural damage assessment for each free-space configuration node, the structural damage assessment being indicative of an assessment of potential damage to at least one critical anatomical area represented by the forbidden configuration nodes, each forbidden configuration node having an infinite structure damage assessment cost,
wherein the free-space configuration nodes include: a safe free-space configuration node having a zero structural damage assessment cost; a risky free-space configuration node having a finite structural damage assessment cost; and a risky free-space configuration node having an infinite structural damage assessment cost,
wherein the path planning comprises evaluating a metric representing a cost for transitioning from one configuration node to a neighboring configuration node, wherein the metric depends on the structural damage assessment costs of the free-space configuration nodes.

14. The system of claim 13, wherein the data processing device (210) is further operable to augment the configuration space node structure as constructed within the data storage medium (220) with parameter values quantifying each node of the configuration space node structure in accordance with a target node and an entry area of the anatomical region (100),
wherein the augmentation of the configuration space node structure includes a geometric expansion of the target node involving at least one free-space configuration node geometrically neighboring the target node serving as a surrogate seed node.

**Patentansprüche**

1. Verfahren (120) zur Planung eines Weges entsprechend einem chirurgischen Eingriff, wobei das Verfahren die folgenden Schritte umfasst, wonach:

   (110) eine Konfigurationsraumknotenstruktur innerhalb eines Datenspeichermediums (220) erzeugt wird, wobei die Konfigurationsraumknotenstruktur Free-Space-Konfigurationsknoten und unzulässige Konfigurationsknoten enthält, die einen diskretisierten Konfigurationsraum eines anatomischen Bereichs (100) eines Körpers darstellen; und

   (S122) eine Strukturschadensfeststellung für jeden Free-Space-Konfigurationsknoten erzeugt wird, wobei die Strukturschadensfeststellung für eine Feststellung potentieller Beschädigung an mindestens einem kritischen anatomischen Areal des anatomischen Bereichs (100), dargestellt durch die unzulässigen Konfigurationsknoten, indikativ ist, wobei jeder unzulässige Konfigurationsknoten infinite Strukturschadensfeststellungskosten hat, wobei die Free-Space-Konfigurationsknoten umfassen: einen sicheren Free-Space-Konfigurationsknoten mit null Strukturschadensfeststellungskosten; wobei ein riskanter Free-Space-Konfigurationsknoten finite Strukturschadensfeststellungskosten hat; und ein riskanter Free-Space-Konfigurationsknoten infinite Strukturschadensfeststellungskosten hat,

   wobei die Wegplanung das Auswerten einer Metrik umfasst, die Kosten zum Übergang von einem Konfigurationsknoten zu einem benachbarten Konfigurationsknoten darstellt, wobei die Metrik von den Strukturschadensfeststellungskosten der Free-Space-Konfigurationsknoten abhängt.

2. Verfahren nach Anspruch 1, wobei das mindestens eine kritische, anatomische Areal mindestens eine(einen) der folgenden enthält:

   eine kritische anatomische Struktur innerhalb des anatomischen Bereichs (100); sowie
   einen fragilen Eintrittspunkt in den anatomischen Bereich (100).

3. Verfahren nach Anspruch 1, wobei die Erzeugung einer Strukturschadensfeststellung für jeden Free-Space-Konfigurationsknoten beinhaltet:

   Erzeugen eines markierten Bildes (142) des anatomischen Bereichs (100) mit mindestens einem unzulässigen Areal des anatomischen Bereichs (100); und
   Berechnen von Strukturschadensfeststellungskosten für jeden dem markierten Bild (142) zugeordneten Free-Space-Konfigurationsknoten.

4. Verfahren nach Anspruch 3, wobei die Erzeugung des markierten Bildes (142) des anatomischen Bereichs (100) beinhaltet:

   das Inübereinstimmungbringen von mindestens einem Patientenbild des anatomischen Bereichs (100) mit einem Atlas (143) des anatomischen Bereichs (100).

5. Verfahren nach Anspruch 3, wobei die Berechnung von Strukturschadensfeststellungskosten für jeden dem markierten Bild (142) zugeordneten Free-Space-Konfigurationsknoten beinhaltet:

   Erzeugen einer Basishinderniskarte (160), die von jedem unzulässigen Areal des anatomischen Bereichs (100), dem markierten Bild (142) zugeordnet, abgeleitet wird, wobei jeder Free-Space-Konfigurationsknoten der Basishinderniskarte (160) Strukturschadensfeststellungskosten von weniger als unendlich aufweist.

6. Verfahren nach Anspruch 5, wobei die Berechnung von Strukturschadensfeststellungskosten für jeden dem markierten Bild (142) zugeordneten Free-Space-Konfigurationsknoten weiterhin beinhaltet:

   Erzeugen einer dilatierten Basishinderniskarte (161), die von mindestens einer um jedes unzulässiges Areal des anatomischen Bereichs (100) erzeugten Sicherheitszone abgeleitet wird, wobei jeder Free-Space-Konfgurationsknoten der dilatierten Basishinderniskarte (161) innerhalb einer der mindestens einen Sicherheitszone infinite Strukturschadensfeststellungskosten aufweist.

7. Verfahren nach Anspruch 3, wobei die Berechnung von Strukturschadensfeststellungskosten für jeden dem markierten Bild (142) zugeordneten Free-Space-Konfigurationsknoten weiterhin umfasst:

Erzeugen einer Hindernisabstandskarte (162), die von einem Abstand zu einem nächsten unzulässigen Areal des anatomischen Bereichs (100) abgeleitet wird.

8. Verfahren nach Anspruch 8, wobei die Berechnung von Strukturschadensfeststellungskosten für jeden dem markierten Bild (142) zugeordneten Free-Space-Konfigurationsknoten weiterhin beinhaltet:

Erzeugen einer empirisch gewichteten Karte (163), die von einer empirischphysikalischen Sensitivität jedes unzulässigen Areals des anatomischen Bereichs (100), dem markierten Bild (142) zugeordnet, abgeleitet wird.

9. Verfahren nach Anspruch 1, wonach weiterhin:

die Konfigurationsraumknotenstruktur, wie innerhalb des Datenspeichermediums (220) erzeugt, mit Strukturschadensfeststellungswerten, die jeden Knoten der Konfigurationsraumknotenstruktur quantifizieren, augmentiert wird.

10. Verfahren nach Anspruch 9, wobei die Augmentation der Konfigurationsraumknotenstruktur eine Kostenfunktion einer Gesamtdistanz kumulativer Übergänge zwischen Knoten entlang dem Weg beinhaltet.

11. Verfahren nach Anspruch 9, wobei die Augmentation der Konfigurationsraumknotenstruktur eine Kostenfunktion eines Instruments beinhaltet, das dem chirurgischen Eingriff unter Einbeziehung von mindestens einer Dimension des Instruments zugeordnet ist.

12. Verfahren nach Anspruch 9, wobei die Augmentation der Konfigurationsraumknotenstruktur eine geometrische Expansion eines Zielknotens unter Einbeziehung von mindestens einem Free-Space-Konfigurationsknoten in geometrischer Angrenzung an den als ein Surrogate-Seed-Knoten dienender Zielknoten beinhaltet.

13. System zur Planung eines Weges entsprechend einem chirurgischen Eingriff, wobei das System umfasst:

ein Datenspeichermedium (220); sowie
eine Datenverarbeitungseinrichtung (210) in elektrischer Verbindung mit dem Datenspeichermedium (220), um eine Konfigurationsraumknotenstruktur innerhalb des Datenspeichermediums (220) zu erzeugen, wobei die Konfigurationsraumknotenstruktur Free-Space-Konfigurationsknoten und unzulässige Konfigurationsknoten enthält, die einen diskretisierten Konfigurationsraum eines anatomischen Bereichs (100) eines Körpers darstellen,
wobei die Datenverarbeitungseinrichtung (210) so arbeitet, dass sie eine Strukturschadensfeststellung für jeden Free-Space-Konfigurationsknoten erzeugt, wobei die Strukturschadensfeststellung für eine Feststellung potentieller Beschädigung an mindestens einem kritischen anatomischen Areal, dargestellt durch die unzulässigen Konfigurationsknoten, indikativ sind, wobei jeder unzulässige Konfigurationsknoten infinite Strukturschadensfeststellungskosten hat,
wobei die Free-Space-Konfigurationsknoten umfassen: einen sicheren Free-Space-Konfigurationsknoten mit null Strukturschadensfeststellungskosten; wobei ein riskanter Free-Space-Konfigurationsknoten finite Strukturschadensfeststellungskosten hat; und ein riskanter Free-Space-Konfigurationsknoten infinite Strukturschadensfeststellungskosten hat,
wobei die Wegplanung das Auswerten einer Metrik umfasst, die Kosten zum Übergang von einem Konfigurationsknoten zu einem benachbarten Konfigurationsknoten darstellt, wobei die Metrik von den Strukturschadensfeststellungskosten der Free-Space-Konfigurationsknoten abhängt.

14. System nach Anspruch 13, wobei die Datenverarbeitungseinrichtung (210) weiterhin so arbeitet, dass sie die Konfigurationsraumknotenstruktur, wie innerhalb des Datenspeichermediums (220) erzeugt, mit Parameterwerten, die jeden Knoten der Konfigurationsraumknotenstruktur entsprechend einem Zielknoten und einem Eintrittsareal des anatomischen Bereichs (100) quantifizieren, augmentiert,
wobei die Augmentation der Konfigurationsraumknotenstruktur eine geometrische Expansion des Zielknotens unter Einbeziehung von mindestens einem Free-Space-Konfigurationsknoten in geometrischer Angrenzung an den als ein Surrogate-Seed-Knoten dienender Zielknoten beinhaltet.

**Revendications**

1. Procédé (120) pour préparer un itinéraire selon une application chirurgicale, le procédé comprenant :

   (110) la construction d'une structure nodale d'un espace de configuration sur un support de stockage de données (220), la structure nodale d'un espace de configuration comprenant des noeuds de configuration sans espace et des noeuds de configuration interdits représentant un espace de configuration discrétisé d'une région anatomique (100) d'un corps ; et
   (S122) la génération d'une évaluation d'un dommage structural pour chaque noeud de configuration sans espace, l'évaluation du dommage structural étant indicative d'une évaluation d'un dommage potentiel dans au moins une zone anatomique critique de la région anatomique (100) représentée par les noeuds de configuration interdits, chaque noeud de configuration interdit ayant un coût d'évaluation du dommage structural infini,
   dans lequel les noeuds de configuration sans espace comprennent : un noeud de configuration sans espace sûr ayant un coût d'évaluation de dommage structural de zéro ; un noeud de configuration sans espace à risque ayant un coût d'évaluation de dommage structural fini ; et un noeud de configuration sans espace à risque ayant un coût d'évaluation de dommage structural infini,
   dans lequel la préparation de l'itinéraire comprend l'évaluation d'une mesure représentant un coût pour le passage d'un noeud de configuration à un noeud de configuration voisin, dans lequel la mesure dépend des coûts d'évaluation de dommage structural des noeuds de configuration sans espace.

2. Procédé selon la revendication 1, dans lequel la au moins une région anatomique comprend au moins l'une parmi :

   une structure anatomique critique dans la région anatomique (100) ; et
   un point d'entrée fragile dans la région anatomique (100).

3. Procédé selon la revendication 1, dans lequel la génération d'une évaluation d'un dommage structural pour chaque noeud de configuration sans espace comprend :

   la génération d'une image marquée (142) de la région anatomique (100) comprenant au moins une région interdite de la région anatomique (100) ; et
   le calcul des coûts d'évaluation de dommage structural pour chaque noeud de configuration sans espace associé à l'image marquée (142).

4. Procédé selon la revendication 3, dans lequel la génération de l'image marquée (142) de la région anatomique (100) comprend :

   l'enregistrement d'au moins une image de patient de la région anatomique (100) avec un atlas (143) de la région anatomique (100).

5. Procédé selon la revendication 3, dans lequel le calcul des coûts d'évaluation de dommage structural pour chaque noeud de configuration sans espace associé à l'image marquée (142) comprend :

   la génération d'une carte d'obstacle de base (160) découlant de chaque zone interdite de la région anatomique (100) associée à l'image marquée (142), chaque noeud de configuration sans espace de la carte d'obstacle de base (160) ayant un coût d'évaluation de dommage structural inférieur à l'infini.

6. Procédé selon la revendication 5, dans lequel le calcul des coûts d'évaluation de dommage structural pour chaque noeud de configuration sans espace associé à l'image marquée (142) comprend en outre :

   la génération d'une carte d'obstacle de base dilatée (161) découlant d'au moins une zone de sécurité établie autour de chaque zone interdite de la région anatomique (100), chaque noeud de configuration sans espace de la carte d'obstacle de base dilatée (161) dans l'une des au moins une zone de sécurité ayant un coût d'évaluation de dommage structural infini.

7. Procédé selon la revendication 3, dans lequel le calcul des coûts d'évaluation de dommage structural pour chaque noeud de configuration sans espace associé à l'image marquée (142) comprend en outre :

   la génération d'une carte de distance d'obstacle (162) découlant d'une distance jusqu'à une zone interdite la plus proche de la région anatomique (100).

**8.** Procédé selon la revendication 3, dans lequel le calcul de coûts d'évaluation de dommage structural pour chaque noeud de configuration sans espace associé à l'image marquée (142) comprend en outre :

la génération d'une carte pondérée empirique (163) découlant d'une sensibilité physique empirique de chaque zone interdite de la région anatomique (100) associée à l'image marquée (142).

**9.** Procédé selon la revendication 1, comprenant en outre :

l'augmentation de la structure nodale de l'espace de configuration construite sur le support de stockage de données (220) avec des valeurs d'évaluation de dommage structural quantifiant chaque noeud de la structure nodale d'espace de configuration.

**10.** Procédé selon la revendication 9, dans lequel l'augmentation de la structure nodale de l'espace de configuration comprend une fonction de coût d'une distance totale de transitions cumulées entre des noeuds le long de l'itinéraire.

**11.** Procédé selon la revendication 9, dans lequel l'augmentation de la structure nodale de l'espace de configuration comprend une fonction de coût d'un instrument associée à l'application chirurgicale impliquant au moins une dimension de l'instrument.

**12.** Procédé selon la revendication 9, dans lequel l'augmentation de la structure nodale de l'espace de configuration comprend une extension géométrique d'un noeud cible impliquant au moins un noeud de configuration sans espace voisin d'un point de vue géométrique du noeud cible servant de noeud d'ensemencement de substitution.

**13.** Système de préparation d'itinéraire selon une application chirurgicale, le système comprenant :

un milieu de stockage de données (220) ; et
un dispositif de traitement des données (210) en communication électrique avec le support de stockage de données (220) pour construire une structure nodale de l'espace de configuration dans le milieu de stockage de données (220), la structure nodale de l'espace de configuration comprenant des noeuds de configuration sans espace et des noeuds de configuration interdits représentant un espace de configuration discrétisé d'une région anatomique (100) d'un corps,
dans lequel le dispositif de traitement des données (210) peut fonctionner de façon à générer une évaluation de dommage structural pour chaque noeud de configuration sans espace, l'évaluation du dommage structural étant indicative d'une évaluation d'un dommage potentiel dans au moins une zone anatomique critique représentée par les noeuds de configuration interdits, chaque noeud de configuration interdit ayant un coût d'évaluation du dommage structural infini,
dans lequel les noeuds de configuration sans espace comprennent : un noeud de configuration sans espace sûr ayant un coût d'évaluation de dommage structural de zéro ; un noeud de configuration sans espace à risque ayant un coût d'évaluation de dommage structural fini ; et un noeud de configuration sans espace à risque ayant un coût d'évaluation de dommage structural infini,
dans lequel la préparation de l'itinéraire comprend l'évaluation d'une mesure représentant un coût pour le passage d'un noeud de configuration à un noeud de configuration voisin, dans lequel la mesure dépend des coûts d'évaluation de dommage structural des noeuds de configuration sans espace.

**14.** Système selon la revendication 13, dans lequel le dispositif de traitement des données (210) peut en outre fonctionner de façon à augmenter la structure nodale de l'espace de configuration construit dans le support de stockage de données (220) avec des valeurs de paramètre quantifiant chaque noeud de la structure nodale de l'espace de configuration selon un noeud cible et une zone d'entrée de la région anatomique (100),
dans lequel l'augmentation de la structure nodale de l'espace de configuration comprend une extension géométrique du noeud cible impliquant au moins un noeud de configuration sans espace voisin d'un point de vue géométrique du noeud cible servant de noeud d'ensemencement de substitution.

| | | | |
|---|---|---|---|
| A | Frontal eye fields | B | Somatosensory |
| C | Broca's | D | motor |
| E | Audition | F | ?? |
| G | Wernicke's | H | Vision |
| I | Cognition | K | Visual-parietal |
| L | Emotion | M | Visual-temporal |

# FIG. 1
(Prior art)

FIG. 2

(Prior art)

FIG. 3

S120 — Flowchart

110 → Setup phase

S121 — Critical area detection

S122 — Structural damage weight computation
(FIG. 6)

- - - - - - - - - - - - - - - - - - - - - - - - - - - - - - - - - - - - - - -

111 → Path planning phase

S123 — Target point / insertion area selections

S124 — Target point propagation
(Geometric expansion) / (Home node expansion)

S125 — Optimal point identification

S126 — Path generation

Terminate

# FIG. 4

FIG. 5
(Prior art)

Original MRI image

Segmented image

140    141

Segmentation

Final labeled
image

Registration

142    143

Atlas

# FIG. 6

S150 — Flowchart

S151 — Base obstacle mapping
(FIG. 9)

S152 — Dilated obstacle mapping
(FIG. 10)

S153 — Obstacle distance mapping
(FIG. 11)

Empirical weighted mapping
(FIG. 12) — S154

S155 — Obstacle map / empirical weighted map combination
(FIG. 13)

Terminate

# FIG. 7

0 — INF

# FIG. 8

160

FIG. 9

161

FIG. 10

FIG. 11

FIG. 12

FIG. 13

S170 — ⬡ Flowchart ⬡

S171 — | Target node identification |

S172 — | Geometric expansion of target node
(FIG. 16) |

( Terminate )

# FIG. 14

180 ↘    181 ↘

⟹ Expansion ⟹

# FIG. 15

FIG. 16

240 241

Sagittal suture

244 245

Lambdoid suture

242 243

246 247

Lambdoid suture

248

Superior and inferior temporal lines

249

250

# FIG. 17
## (Prior art)

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007042986 A2
- EP 1844725 A1
- WO 2008032230 A
- WO 2007042986 A

**Non-patent literature cited in the description**

- **KAREN I. TROVATO.** A* Planning in Discrete Configuration Spaces of Autonomous System. University of Amsterdam, 1996
- **LANCASTER JL ; WOLDORFF MG ; PARSONS LM ; LIOTTI M ; FREITAS CS ; RAINEY L ; KOCHUNOV PV ; NICKERSON D ; MIKITEN SA ; FOX PT.** Automated Talairach Atlas labels for functional brain mapping. *Human Brain Mapping,* 2000, vol. 10, 120-131
- **SEKHAR ; FESSLER.** Atlas of Neurosurgical Techniques. 422-429